(19) Europäisches Patentamt
European Patent Office
Office européen des brevets

(11) EP 0 728 465 B1

(12) **FASCICULE DE BREVET EUROPEEN**

(45) Date de publication et mention de la délivrance du brevet:
**19.03.1997 Bulletin 1997/12**

(51) Int. Cl.$^6$: **A61K 7/13**

(21) Numéro de dépôt: **96400185.3**

(22) Date de dépôt: **25.01.1996**

(54) **Composition de teinture d'oxydation des fibres kératiniques comprenant un méta-aminophénol et la 6-hydroxyindoline et procédé de teinture mettant en oeuvre cette composition**

Oxidationshaarfärbemittel, enthaltend ein Meta-Aminophenol und 6-Hydroxyindolin, und Färbeverfahren mit demselben Mittel

Composition for dyeing keratinous fibers comprising a meta-aminophenol and 6-hydroxyindoline, and dyeing process using such a composition

(84) Etats contractants désignés:
**DE ES FR GB IT**

(30) Priorité: **27.02.1995 FR 9502272**

(43) Date de publication de la demande:
**28.08.1996 Bulletin 1996/35**

(73) Titulaire: **L'OREAL**
**75008 Paris (FR)**

(72) Inventeur: **Maubru, Mireille**
**F-78400 Chatou (FR)**

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**WO-A-93/09759**
**DE-A- 1 916 139**
**GB-A- 2 180 215**

EP 0 728 465 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.13.17/3.4

## Description

La présente invention a pour objet une composition pour la teinture d'oxydation des fibres kératiniques, en particulier des fibres kératiniques humaines telles que les cheveux, comprenant au moins une base d'oxydation convenablement sélectionnée, au moins un méta-aminophénol à titre de premier coupleur et de la 6-hydroxyindoline et/ou au moins l'un de ses sels d'addition avec un acide à titre de deuxième coupleur, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés indoliques ou indoliniques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Pour la réalisation de nuances naturelles ou à reflets, il est courant d'utiliser des compositions tinctoriales comprenant un dérivé de paraphénylènediamine à titre de base d'oxydation et un dérivé de méta-aminophénol à titre de coupleur. Les colorations obtenues avec de telles compositions tinctoriales ne sont cependant pas entièrement satisfaisantes car elles présentent généralement une forte sélectivité.

D'autre part, il a déjà été proposé, notamment dans la demande de brevet FR 2 008 797, des compositions pour la teinture d'oxydation en milieu alcalin des fibres kératiniques comprenant au moins une base d'oxydation telle que par exemple la paraphénylènediamine ou un dérivé de paraphénylènediamine, en association avec un coupleur indolinique comme par exemple la 6-hydroxyindoline. Ces compositions ne sont pas non plus entièrement satisfaisantes car elles conduisent à des colorations présentant également une forte sélectivité.

Or, la demanderesse vient maintenant de découvrir qu'il est possible d'obtenir de nouvelles teintures en milieu acide, neutre ou alcalin, capables d'engendrer des colorations puissantes et moins sélectives que les colorations de l'art antérieur, et résistant bien aux diverses agressions que peuvent subir les cheveux, en associant au moins une base d'oxydation convenablement sélectionnée, au moins un méta-aminophénol à titre de premier coupleur et de la 6-hydroxyindoline et/ou au moins l'un de ses sels d'addition avec un acide à titre de second coupleur.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation choisie parmi les paraphénylènediamines et/ou les bis-phénylalkylènediamines,
- au moins un méta-aminophénol à titre de premier coupleur,
- au moins un second coupleur choisi parmi la 6-hydroxyindoline et ses sels d'addition avec un acide.

La composition de teinture d'oxydation conforme à l'invention permet d'obtenir des colorations puissantes aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes).

L'invention a également pour objet un procédé de teinture d'oxydation des fibres kératiniques mettant en oeuvre cette composition.

Parmi les méta-aminophénols utilisables à titre de premier coupleur dans les compositions conformes à l'invention, on peut citer les composés répondant à la formule (I) suivante, et leurs sels d'addition avec un acide :

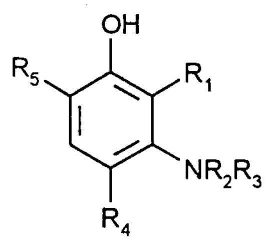

(I)

dans laquelle :

$R_1$ et $R_4$, identiques ou différents, représentent un atome d'hydrogène ou d'halogène, un radical alkyle en $C_1$-$C_4$ ou alcoxy en $C_1$-$C_4$,
$R_2$ représente un atome d'hydrogène, un radical alkyle en $C_1$-$C_4$, monohydroxyalkyle en $C_1$-$C_4$, polyhydroxyalkyle en $C_2$-$C_4$, trifluoroalkyle en $C_1$-$C_4$ ou carbamoylméthyle,
$R_3$ représente un atome d'hydrogène, un radical alkyle en $C_1$-$C_4$ ou bien encore forme, avec $R_2$ et l'atome d'azote, un hétérocycle à 5 ou 6 sommets,
$R_5$ représente un atome d'hydrogène ou d'halogène, un radical alkyle en $C_1$-$C_4$, alcoxy en $C_1$-$C_4$, monohydroxyalkyle en $C_1$-$C_4$ ou polyhydroxyalkyle en $C_2$-$C_4$.

Parmi les méta-aminophénols de formule (I) ci-dessus, utilisés à titre de premier coupleur dans les compositions tinctoriales conformes à l'invention, on peut plus particulièrement citer le 3-amino phénol, le 5-amino 2-méthoxy phénol, le 5-amino 4-chloro 2-méthyl phénol, le 5-amino 2,4-diméthoxy phénol, le 5-amino 2-β-hydroxyéthyloxy phénol, le 5-N-(β-hydroxyéthyl)amino 4-méthoxy 2-méthyl phénol, le 5-amino 4-méthoxy 2-méthyl phénol, le 5-N-(γ-hydroxypropyl)amino 2-méthyl phénol, le 5-N-(β-hydroxyéthyl)amino 2-méthyl phénol, le 5-amino 2-méthyl phénol, le 3-N-(carbamoylméthyl)amino phénol, le 5-N-(carbamoylméthyl)amino 2-méthyl phénol, le 3-N,N-(diméthyl)amino phénol, le 3-N,N-(diéthyl)amino phénol, le 3-amino 2,4-dichloro phénol, le 3-amino 4,6-dichloro phénol, le 5-amino 6-chloro 2-méthyl phénol, le 2-chloro 5-N-(2',2',2'-trifluoroéthyl)amino phénol, le 5-amino 4-chloro 2-méthyl phénol, le 3-N-(cyclopentyl)amino phénol, et leurs sels d'addition avec un acide.

Parmi les paraphénylènediamines utilisables à titre de base d'oxydation dans les compositions conformes à l'invention, on peut citer les composés répondant à la formule (II) suivante, et leurs sels d'addition avec un acide :

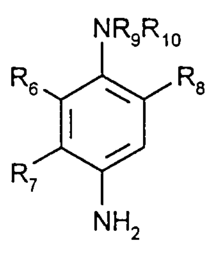

(II)

dans laquelle :

$R_6$, $R_7$ et $R_8$, identiques ou différents, représentent un atome d'hydrogène ou monohydroxyalkyle en $C_1$-$C_4$ ou polyhydroxyalkyle en $C_2$-$C_4$, d'halogène, un radical alkyle en $C_1$-$C_4$, alcoxy en $C_1$-$C_4$, sulfo, carboxy,
$R_9$ et $R_{10}$, identiques ou différents, représentent un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), carbamylalkyle en $C_1$-$C_4$, mésylaminoalkyle en $C_1$-$C_4$, acétylaminoalkyle en $C_1$-$C_4$, uréidoalkyle en $C_1$-$C_4$, carbalcoxy($C_1$-$C_4$)aminoalkyle($C_1$-$C_4$), sulfoalkyle en $C_1$-$C_4$, pipéridinoalkyle en $C_1$-$C_4$, morpholinoalkyle en $C_1$-$C_4$, phényle ou phényle substitué en position para par un groupement amino, ou bien encore $R_9$ et $R_{10}$ forment, conjointement avec l'atome d'azote auquel ils sont liés, un hétérocycle pipéridino ou morpholino ;
étant entendu que si $R_9$ et $R_{10}$ ne représentent pas simultanément un atome d'hydrogène, alors un au moins des radicaux $R_6$, $R_8$ doit représenter un atome d'hydrogène.

Parmi les radicaux alkyle en $C_1$-$C_4$ et alcoxy en $C_1$-$C_4$ des formules (I) et (II) ci-dessus, on peut citer notamment

les radicaux méthyle, éthyle, propyle, méthyloxy et éthyloxy.

Parmi les paraphénylènediamines de formule (II) ci-dessus, utilisées à titre de base d'oxydation dans les compositions tinctoriales conformes à l'invention, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la 2-méthyl 5-méthoxy paraphénylènediamine, la 2,6-diméthyl 5-méthoxy paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 4-amino N,N-(éthyl, carbamylméthyl) aniline, la 4-amino 3-méthyl N,N-(éthyl, carbamylméthyl) aniline, la 4-amino N,N-(éthyl, β-pipéridinoéthyl) aniline, la 4-amino 3-méthyl N,N-(éthyl, β-pipéridinoéthyl) aniline, la 4-amino N,N-(éthyl, β-morpholinoéthyl) aniline, la 4-amino 3-méthyl N,N-(éthyl, β-morpholinoéthyl) aniline, la 4-amino N,N-(éthyl, β-acétylaminoéthyl) aniline, la 4-amino N-(β-méthoxyéthyl) aniline, la 4-amino 3-méthyl N,N-(éthyl, β-acétylaminoéthyl) aniline, la 4-amino N,N-(éthyl, β-mésylaminoéthyl) aniline, la 4-amino 3-méthyl N,N-(éthyl, β-mésylaminoéthyl) aniline, la 4-amino N,N-(éthyl, β-sulfoéthyl) aniline, la 4-amino 3-méthyl N,N-(éthyl, β-sulfoéthyl) aniline, la N-[(4'-amino)phényl] morpholine, la N-[(4'-amino)phényl] pipéridine, la 2-β-hydroxyéthyl paraphénylènediamine, la 2-fluoro paraphénylènediamine, la 2-carboxy paraphénylènediamine, la 2-sulfo paraphénylènediamine, la 2-isopropyl paraphénylènediamine, la 2-n-propyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, et leurs sels d'addition avec un acide.

Parmi les paraphénylènediamines de formule (II) ci-dessus, utilisées à titre de base d'oxydation dans les compositions tinctoriales conformes à l'invention, sont plus particulièrement préférées la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, et leurs sels d'addition avec un acide.

Parmi les bis-phénylalkylènediamines utilisables à titre de base d'oxydation dans les compositions tinctoriales conformes à l'invention, on peut plus particulièrement citer les composés répondant à la formule (III) suivante, et leurs sels d'addition avec un acide :

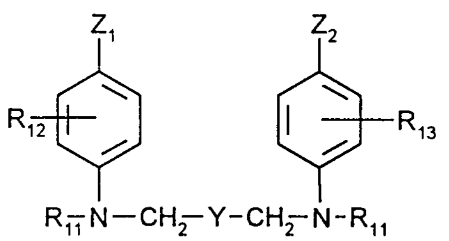

(III)

dans laquelle :

$Z_1$ et $Z_2$, identiques ou différents, représentent un radical hydroxyle ou $NHR_{14}$ dans lequel $R_{14}$ représente un atome d'hydrogène ou un radical alkyle en $C_1$-$C_4$,

$R_{11}$ représente un atome d'hydrogène, un radical alkyle en $C_1$-$C_4$, monohydroxyalkyle en $C_1$-$C_4$, polyhydroxyalkyle en $C_2$-$C_4$ ou aminoalkyle en $C_1$-$C_4$ dont le reste amino peut être substitué,

$R_{12}$ et $R_{13}$, identiques ou différents, représentent un atome d'hydrogène ou d'halogène ou un radical alkyle en $C_1$-$C_4$,

Y représente un radical pris dans le groupe constitué par les radicaux suivants :

$-(CH_2)_n-$; $-(CH_2)_m-O-(CH_2)_m-$; $-(CH_2)_m-CHOH-(CH_2)_m-$ et

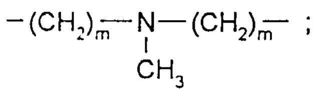

dans lesquels n est un nombre entier compris entre 0 et 8 inclusivement et m est un nombre entier compris entre 0 et 4 inclusivement.

Parmi les radicaux alkyle en $C_1$-$C_4$ de la formule (III) ci-dessus, on peut citer notamment les radicaux méthyle, éthyle et propyle.

Parmi les bis-phénylalkylènediamines de formules (III) ci-dessus, utilisées à titre de base d'oxydation dans les compositions tinctoriales conformes à l'invention, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol est particulièrement préféré.

Les sels d'addition avec un acide utilisables dans le cadre des compositions tinctoriales de l'invention sont notamment choisis parmi les chlorhydrates, les bromhydrates, les sulfates et les tartrates.

La ou les bases d'oxydation conformes à l'invention, c'est à dire la ou les paraphénylènediamines et/ou la ou les bis-phénylalkylènediamines, représentent de préférence de 0,0005 à 12 % en poids environ du poids total de la composition tinctoriale, et encore plus préférentiellement de 0,005 à 6 % en poids environ de ce poids.

Le ou les méta-aminophénols de formule (I), utilisés à titre de premier coupleur dans les compositions tinctoriales conformes à l'invention, représentent de préférence de 0,0001 à 10 % en poids environ du poids total de la composition tinctoriale et encore plus préférentiellement de 0,005 à 5 % en poids environ de ce poids.

La 6-hydroxyindoline et/ou son ou ses sels d'addition avec un acide, utilisés à titre de second coupleur dans les compositions tinctoriales conformes à l'invention, représentent de préférence de 0,0001 à 5 % en poids environ du poids total de la composition tinctoriale et encore plus préférentiellement de 0,005 à 3 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante :

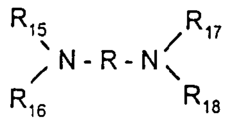

(IV)

dans laquelle R est un reste propylène éventuellement substitué par un groupement hydroxyle ou un radical alkyle en $C_1$-$C_4$ ; $R_{15}$, $R_{16}$, $R_{17}$ et $R_{18}$, identiques ou différents, représentent un atome d'hydrogène, un radical alkyle en $C_1$-$C_4$ ou hydroxyalkyle en $C_1$-$C_4$.

La composition tinctoriale conforme à l'invention peut encore contenir, en plus des colorants définis ci-dessus, d'autres bases d'oxydation et/ou d'autres coupleurs et/ou des colorants directs, notamment pour modifier les nuances ou les enrichir en reflets.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à l'association ternaire conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

On a réalisé les compositions tinctoriales 1 à 5 suivantes :

| COMPOSITION | 1 (*) | 2 (*) | 3 (*) | 4 (**) | 5 (**) |
|---|---|---|---|---|---|
| Paraphénylènediamine (en moles) | $3.10^{-3}$ | $3.10^{-3}$ | $3.10^{-3}$ | $3.10^{-3}$ | $3.10^{-3}$ |
| 3-amino phénol (en moles) | $3.10^{-3}$ | | | $1{,}5.10^{-3}$ | |
| 5-amino 2-méthyl phénol (en moles) | | $3.10^{-3}$ | | | $1{,}5.10^{-3}$ |
| Chlorhydrate de 6-hydroxyindoline (en moles) | | | $3.10^{-3}$ | $1{,}5.10^{-3}$ | $1{,}5.10^{-3}$ |
| Support de teinture commun | (***) | (***) | (***) | (***) | (***) |
| Eau déminéralisée q.s.p. | 100 g | 100 g | 100 g | 100 g | 100 g |

(*) : composition tinctoriale ne faisant pas partie de l'invention

(**) : composition tinctoriale conforme à l'invention

(***) : Support de teinture commun :

| | | |
|---|---|---|
| - Alcool oléique polyglycérolé à 2 moles de glycérol | 4,0 | g |
| - Alcool oléique polyglycérolé à 4 moles de glycérol, à 78 % de matières actives (M.A.) | 5,69 | g M.A. |
| - Acide oléique | 3,0 | g |
| - Amine oléique à 2 moles d'oxyde d'éthylène vendue sous la dénomination commerciale ETHOMEEN O12 par la société AKZO | 7,0 | g |
| - Laurylamino succinamate de diéthylaminopropyle, sel de sodium, à 55 % de M.A. | 3,0 | g M.A. |
| - Alcool oléique | 5,0 | g |
| - Diéthanolamide d'acide oléique | 12,0 | g |
| - Propylèneglycol | 3,5 | g |

- Alcool éthylique 7,0 g
- Dipropylèneglycol 0,5 g
- Monométhyléther de propylèneglycol 9,0 g
- Métabisulfite de sodium en solution aqueuse, à 35 % de M.A. 0,455 g M.A.
- Acétate d'ammonium 0,8 g
- Antioxydant, séquestrant q.s.
- Parfum, conservateur q.s.
- Ammoniaque à 20 % de $NH_3$ 10,0 g

Au moment de l'emploi, on a mélangé chaque composition tinctoriale 1 à 5 avec une quantité égale en poids d'une solution d'eau oxygénée à 20 volumes (6 % en poids).

Chaque mélange résultant a été appliqué pendant 30 minutes, d'une part sur des mèches de cheveux gris naturels à 90 % de blancs (mèche n°1 de cheveux non sensibilisés) et d'autre part sur une mèche de ces mêmes cheveux gris à 90 % de blancs mais ayant subi une permanente (mèche n° 2 de cheveux sensibilisés). Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur entre deux mèches est calculée en appliquant la formule de NICKERSON : $\Delta E = 0{,}4\ C_0 \Delta H + 6 \Delta V + 3\ \Delta C$ , telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et $C_0$ représente la pureté de la mèche par rapport à laquelle on désire évaluer la différence de couleur.

La différence de couleur entre cheveux naturels (non sensibilisés) et cheveux permanentés (sensibilisés) a permis de mettre en évidence la sélectivité de chacune des compositions tinctoriales 1 à 5 et a été calculée en appliquant la formule de NICKERSON.

Les résultats figurent dans le tableau ci-dessous :

| EXEMPLE (COMPOSITION) | Couleur sur cheveux naturels | Couleur sur cheveux permanentés | Différence de couleur (sélectivité) | | | |
|---|---|---|---|---|---|---|
| | | | ΔH | ΔV | ΔC | **ΔE** |
| 1 (1) | 8,2 RP 2,7 / 1,6 | 0,8 R 2,3 / 1,3 | 2,6 | 0,4 | 0,3 | **5,0** |
| 2 (2) | 8,3 RP 3,0 / 3,3 | 8,8 RP 2,5 / 3,8 | 0,5 | 0,5 | 0,5 | **5,2** |
| 3 (3) | 4,6 R 3,1 / 1,4 | 7,8 RP 2,8 / 1,6 | 6,8 | 0,3 | 0,2 | **6,2** |
| 4 (4) | 0,9 R 2,9 / 1,5 | 1,3 R 2,7 / 1,5 | 0,4 | 0,2 | 0 | **1,4** |
| 5 (5) | 9,9 RP 2,9 / 2,0 | 9,8 RP 2,5 / 2,0 | 0,1 | 0,4 | 0 | **2,5** |

Ces résultats montrent clairement que les compositions tinctoriales 4 et 5 conformes à l'invention, c'est à dire comprenant une base d'oxydation (paraphénylènediamine), en association avec un méta-aminophénol à titre de premier coupleur et du chlorhydrate de 6-hydroxyindoline à titre de second coupleur, conduisent à des colorations très peu sélectives. Par contre, les compositions tinctoriales comparatives des exemples 1 à 3 (qui ne font pas partie de l'invention, car elles ne contiennent qu'un seul des deux coupleurs conduisent à des colorations nettement plus sélectives.

**Revendications**

1. Composition pour la teinture d'oxydation des fibres kératiniques et en particulier des fibres kératiniques humaines telles que les cheveux, caractérisée par le fait qu'elle comprend, dans un milieu approprié pour la teinture :

   - au moins une base d'oxydation choisie parmi les paraphénylènediamines et/ou les bis-phénylalkylènediamines,
   - au moins un méta-aminophénol à titre de premier coupleur,
   - au moins un second coupleur choisi parmi la 6-hydroxyindoline et ses sels d'addition avec un acide.

2. Composition selon la revendication 1, caractérisée par le fait que le (ou les) méta-aminophénol(s) utilisé(s) à titre de premier coupleur est (sont) choisi(s) parmi les composés répondant à la formule (I) suivante, et leurs sels d'addition avec un acide :

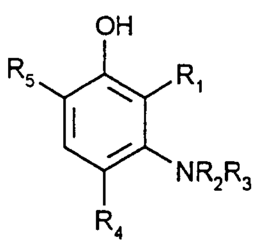

(I)

   dans laquelle :

   $R_1$ et $R_4$, identiques ou différents, représentent un atome d'hydrogène ou d'halogène, un radical alkyle en $C_1$-$C_4$ ou alcoxy en $C_1$-$C_4$,
   $R_2$ représente un atome d'hydrogène, un radical alkyle en $C_1$-$C_4$, monohydroxyalkyle en $C_1$-$C_4$, polyhydroxyalkyle en $C_2$-$C_4$, trifluoroalkyle en $C_1$-$C_4$ ou carbamoylméthyle,
   $R_3$ représente un atome d'hydrogène, un radical alkyle en $C_1$-$C_4$ ou bien encore forme, avec $R_2$ et l'atome d'azote, un hétérocycle à 5 ou 6 sommets,
   $R_5$ représente un atome d'hydrogène ou d'halogène, un radical alkyle en $C_1$-$C_4$, alcoxy en $C_1$-$C_4$, monohydroxyalkyle en $C_1$-$C_4$ ou polyhydroxyalkyle en $C_2$-$C_4$.

3. Composition selon la revendication 2, caractérisée par le fait que le (ou les) méta-aminophénol(s) utilisé(s) à titre de premier coupleur est (sont) choisi(s) parmi le 3-amino phénol, le 5-amino 2-méthoxy phénol, le 5-amino 4-chloro 2-méthyl phénol, le 5-amino 2,4-diméthoxy phénol, le 5-amino 2-β-hydroxyéthyloxy phénol, le 5-N-(β-hydroxyéthyl)amino 4-méthoxy 2-méthyl phénol, le 5-amino 4-méthoxy 2-méthyl phénol, le 5-N-(γ-hydroxypropyl)amino 2-méthyl phénol, le 5-N-(β-hydroxyéthyl)amino 2-méthyl phénol, le 5-amino 2-méthyl phénol, le 3-N-(carbamoylméthyl)amino phénol, le 5-N-(carbamoylméthyl)amino 2-méthyl phénol, le 3-N,N-(diméthyl)amino phénol, le 3-N,N-(diéthyl)amino phénol, le 3-amino 2,4-dichloro phénol, le 3-amino 4,6-dichloro phénol, le 5-amino 6-chloro 2-méthyl phénol, le 2-chloro 5-N-(2',2',2'-trifluoroéthyl)amino phénol, le 5-amino 4-chloro 2-méthyl phénol, le 3-N-(cyclopentyl)amino phénol, et leurs sels d'addition avec un acide.

4. Composition selon l'une quelconque des revendications précédentes, caractérisée par le fait que les paraphénylènediamines sont choisies parmi les composés de formule (II) suivante, et leurs sels d'addition avec un acide :

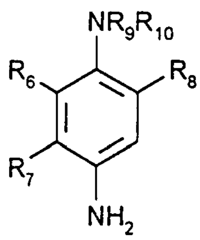

(II)

dans laquelle :

$R_6$, $R_7$ et $R_8$, identiques ou différents, représentent un atome d'hydrogène ou d'halogène, un radical alkyle en $C_1$-$C_4$, alcoxy en $C_1$-$C_4$, sulfo, carboxy, monohydroxyalkyle en $C_1$-$C_4$ ou polyhydroxyalkyle en $C_2$-$C_4$,
$R_9$ et $R_{10}$, identiques ou différents, représentent un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), carbamylalkyle en $C_1$-$C_4$, mésylaminoalkyle en $C_1$-$C_4$, acétylaminoalkyle en $C_1$-$C_4$, uréidoalkyle en $C_1$-$C_4$, carbalcoxy($C_1$-$C_4$)aminoalkyle($C_1$-$C_4$), sulfoalkyle en $C_1$-$C_4$, pipéridinoalkyle en $C_1$-$C_4$, morpholinoalkyle en $C_1$-$C_4$, phényle ou phényle substitué en position para par un groupement amino, ou bien encore $R_9$ et $R_{10}$ forment, conjointement avec l'atome d'azote auquel ils sont liés, un hétérocycle pipéridino ou morpholino ;
étant entendu que si $R_9$ et $R_{10}$ ne représentent pas simultanément un atome d'hydrogène, alors un au moins des radicaux $R_6$, $R_8$ doit représenter un atome d'hydrogène.

**5.** Composition selon la revendication 4, caractérisée par le fait que les paraphénylènediamines de formule (II) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la 2-méthyl 5-méthoxy paraphénylènediamine, la 2,6-diméthyl 5-méthoxy paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 4-amino N,N-(éthyl, carbamylméthyl) aniline, la 4-amino 3-méthyl N,N-(éthyl, carbamylméthyl) aniline, la 4-amino N,N-(éthyl, β-pipéridinoéthyl) aniline, la 4-amino 3-méthyl N,N-(éthyl, β-pipéridinoéthyl) aniline, la 4-amino N,N-(éthyl, β-morpholinoéthyl) aniline, la 4-amino 3-méthyl N,N-(éthyl, β-morpholinoéthyl) aniline, la 4-amino N,N-(éthyl, β-acétylaminoéthyl) aniline, la 4-amino N-(β-méthoxyéthyl) aniline, la 4-amino 3-méthyl N,N-(éthyl, β-acétylaminoéthyl) aniline, la 4-amino N,N-(éthyl, β-mésylaminoéthyl) aniline, la 4-amino 3-méthyl N,N-(éthyl, β-mésylaminoéthyl) aniline, la 4-amino N,N-(éthyl, β-sulfoéthyl) aniline, la 4-amino 3-méthyl N,N-(éthyl, β-sulfoéthyl) aniline, la N-[(4'-amino)phényl] morpholine, la N-[(4'-amino)phényl] pipéridine, la 2-β-hydroxyéthyl paraphénylènediamine, la 2-fluoro paraphénylènediamine, la 2-carboxy paraphénylènediamine, la 2-sulfo paraphénylènediamine, la 2-isopropyl paraphénylènediamine, la 2-n-propyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, et leurs sels d'addition avec un acide.

**6.** Composition selon la revendication 5, caractérisée par le fait que les paraphénylènediamines de formule (II) sont choisies parmi la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N, N-bis-(β-hydroxyéthyl) paraphénylènediamine, la 4-amino N-(β-méthoxyéthyl) aniline, et leurs sels d'addition avec un acide.

**7.** Composition selon l'une quelconque des revendications précédentes, caractérisée par le fait que les bis-phénylalkylènediamines sont choisies parmi les composés de formule (III) suivante, et leurs sels d'addition avec un acide :

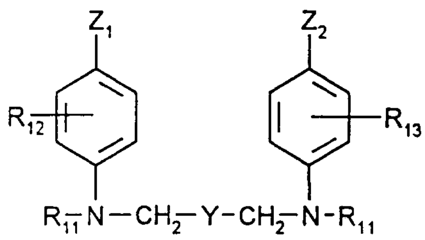 (III)

dans laquelle :

$Z_1$ et $Z_2$, identiques ou différents, représentent un radical hydroxyle ou $NHR_{14}$ dans lequel $R_{14}$ représente un atome d'hydrogène ou un radical alkyle en $C_1$-$C_4$,
$R_{11}$ représente un atome d'hydrogène, un radical alkyle en $C_1$-$C_4$, monohydroxyalkyle en $C_1$-$C_4$, polyhydroxyalkyle en $C_2$-$C_4$ ou aminoalkyle en $C_1$-$C_4$ dont le reste amino peut être substitué,
$R_{12}$ et $R_{13}$, identiques ou différents, représentent un atome d'hydrogène ou d'halogène ou un radical alkyle en $C_1$-$C_4$,
Y représente un radical pris dans le groupe constitué par les radicaux suivants : $-(CH_2)_n-$ ; $-(CH_2)_m-O-(CH_2)_m-$ ; $-(CH_2)_m-CHOH-(CH_2)_m-$ et

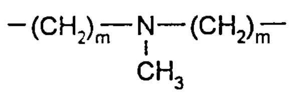 ;

dans lesquels n est un nombre entier compris entre 0 et 8 inclusivement et m est un nombre entier compris entre 0 et 4 inclusivement.

**8.** Composition selon la revendication 7, caractérisée par le fait que les bis-phénylalkylènediamines de formules (III) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

**9.** Composition selon l'une quelconque des revendications précédentes, caractérisée par le fait que les sels d'addition avec un acide sont choisis parmi les chlorhydrates, les bromhydrates, les sulfates et les tartrates.

**10.** Composition selon l'une quelconque des revendications précédentes, caractérisée par le fait que la ou les paraphénylènediamines et/ou la ou les bis-phénylalkylènediamines représentent de 0,0005 à 12 % en poids du poids total de la composition tinctoriale.

**11.** Composition selon la revendication 10, caractérisée par le fait que la ou les paraphénylènediamines et/ou la ou les bis-phénylalkylènediamines représentent de 0,005 à 6 % en poids du poids total de la composition tinctoriale.

**12.** Composition selon l'une quelconque des revendications précédentes, caractérisée par le fait que le ou les méta-aminophénols de formule (I) représentent de 0,0001 à 10 % en poids du poids total de la composition tinctoriale.

**13.** Composition selon la revendication 12, caractérisée par le fait que le ou les méta-aminophénols de formule (I) représentent de 0,005 à 5 % en poids du poids total de la composition tinctoriale.

**14.** Composition selon l'une quelconque des revendications précédentes, caractérisée par le fait que la 6-hydroxyindoline et/ou son ou ses sels d'addition avec un acide représentent de 0,0001 à 5 % en poids du poids total de la composition tinctoriale.

**15.** Composition selon la revendication 14, caractérisée par le fait que la 6-hydroxyindoline et/ou son ou ses sels

d'addition avec un acide représentent de 0,005 à 3 % en poids du poids total de la composition tinctoriale.

**16.** Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**17.** Composition selon l'une quelconque des revendications précédentes, caractérisée par le fait qu'elle présente un pH compris entre 3 et 12.

**18.** Procédé de teinture des fibres kératiniques et en particulier des fibres kératiniques humaines telles que les cheveux caractérisé par le fait que l'on applique sur ces fibres au moins une composition tinctoriale telle que définie à l'une quelconque des revendications 1 à 17 et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

**19.** Procédé selon la revendication 18, caractérisé par le fait que l'agent oxydant est choisi parmi le peroxyde d'hydrogène, le peroxyde d'urée, les bromates de métaux alcalins, les persels tels que les perborates et persulfates.

**20.** Dispositif à plusieurs compartiments, ou "kit" de teinture à plusieurs compartiments, dont un premier compartiment renferme une composition tinctoriale telle que définie à l'une quelconque des revendications 1 à 17 et un second compartiment renferme une composition oxydante.

**Claims**

**1.** Composition for the oxidation dyeing of keratin fibres, and in particular human keratin fibres such as the hair, characterized in that it comprises, in a medium which is suitable for dyeing:

- at least one oxidation base chosen from para-phenylenediamines and/or bis(phenyl)alkylenediamines,
- at least one meta-aminophenol as first coupler,
- at least one second coupler chosen from 6-hydroxyindoline and the addition salts thereof with an acid.

**2.** Composition according to Claim 1, characterized in that the meta-aminophenol(s) used as first coupler is (are) chosen from the compounds corresponding to the following formula (I), and the addition salts thereof with an acid:

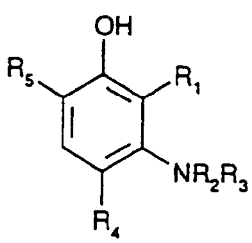

(I)

in which:

$R_1$ and $R_4$, which may be identical or different, represent a hydrogen or halogen atom or a $C_1$-$C_4$ alkyl or $C_1$-$C_4$ alkoxy radical,
$R_2$ represents a hydrogen atom or a $C_1$-$C_4$ alkyl, $C_1$-$C_4$ monohydroxyalkyl, $C_2$-$C_4$ polyhydroxyalkyl, $C_1$-$C_4$ trifluoroalkyl or carbamoylmethyl radical,
$R_3$ represents a hydrogen atom or a $C_1$-$C_4$ alkyl radical or alternatively forms, with $R_2$ and the nitrogen atom, a 5- or 6-membered heterocycle,
$R_5$ represents a hydrogen or halogen atom or a $C_1$-$C_4$ alkyl, $C_1$-$C_4$ alkoxy, $C_1$-$C_4$ monohydroxyalkyl or $C_2$-$C_4$ polyhydroxyalkyl radical.

3. Composition according to Claim 2, characterized in that the meta-aminophenol(s) used as first coupler is (are) chosen from 3-aminophenol, 5-amino-2-methoxyphenol, 5-amino-4-chloro-2-methylphenol, 5-amino-2,4-dimethoxyphenol, 5-amino-2-β-hydroxyethyloxyphenol, 5-N-(β-hydroxyethyl)amino-4-methoxy-2-methylphenol, 5-amino-4-methoxy-2-methylphenol, 5-N-(γ-hydroxypropyl)amino-2-methylphenol, 5-N-(β-hydroxyethyl)amino-2-methylphenol, 5-amino-2-methylphenol, 3-N-(carbamoylmethyl)aminophenol, 5-N-(carbamoylmethyl)amino-2-methylphenol, 3-N,N-(dimethyl)aminophenol, 3-N,N-(diethyl)aminophenol, 3-amino-2,4-dichlorophenol, 3-amino-4,6-dichlorophenol, 5-amino-6-chloro-2-methylphenol, 2-chloro-5-N-(2',2',2'-trifluoroethyl)aminophenol, 5-amino-4-chloro-2-methylphenol and 3-N-(cyclopentyl)aminophenol, and the addition salts thereof with an acid.

4. Composition according to any one of the preceding claims, characterized in that the para-phenylenediamines are chosen from the compounds of following formula (II), and the addition salts thereof with an acid:

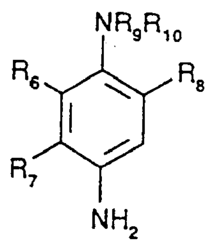

(II)

in which:

$R_6$, $R_7$ and $R_8$, which may be identical or different, represent a hydrogen or halogen atom or a $C_1$-$C_4$ alkyl, $C_1$-$C_4$ alkoxy, sulpho, carboxyl, $C_1$-$C_4$ monohydroxyalkyl or $C_2$-$C_4$ polyhydroxyalkyl radical,
$R_9$ and $R_{10}$, which may be identical or different, represent a hydrogen atom, a $C_1$-$C_4$ alkyl, $C_1$-$C_4$ monohydroxyalkyl, $C_2$-$C_4$ polyhydroxyalkyl, ($C_1$-$C_4$)alkoxy($C_1$-$C_4$)alkyl, $C_1$-$C_4$ carbamylalkyl, $C_1$-$C_4$ mesylaminoalkyl, $C_1$-$C_4$ acetylaminoalkyl, $C_1$-$C_4$ ureidoalkyl, ($C_1$-$C_4$)carbalkoxy($C_1$-$C_4$)aminoalkyl, $C_1$-$C_4$ sulphoalkyl, $C_1$-$C_4$ piperidinoalkyl, $C_1$-$C_4$ morpholinoalkyl or phenyl radical or a phenyl radical substituted in the para position with an amino group, or alternatively $R_9$ and $R_{10}$ form, together with the nitrogen atom to which they are attached, a piperidino or morpholino heterocycle;
it being understood that if $R_9$ and $R_{10}$ do not simultaneously represent a hydrogen atom, then at least one of the radicals $R_6$ and $R_8$ must represent a hydrogen atom.

5. Composition according to Claim 4, characterized in that the para-phenylenediamines of formula (II) are chosen from para-phenylenediamine, para-toluylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)para-phenylenediamine, 4-amino-N,N-bis(β-hydroxyethyl)-3-methylaniline, 4-amino-3-chloro-N,N-bis(β-hydroxyethyl)aniline, 4-amino-N-ethyl-N-(carbamylmethyl)aniline, 4-amino-3-methyl-N-ethyl-N-(carbamylmethyl)aniline, 4-amino-N-ethyl-N-(β-piperidinoethyl)aniline, 4-amino-3-methyl-N-ethyl-N-(β-piperidinoethyl)aniline, 4-amino-N-ethyl-N-(β-morpholinoethyl)aniline, 4-amino-3-methyl-N-ethyl-N-(β-morpholinoethyl)aniline, 4-amino-N-ethyl-N-(β-acetylaminoethyl)aniline, 4-amino-N-(β-methoxyethyl)aniline, 4-amino-3-methyl-N-ethyl-N-(β-acetylaminoethyl)aniline, 4-amino-N-ethyl-N-(β-mesylaminoethyl)aniline, 4-amino-3-methyl-N-ethyl-N-(β-mesylaminoethyl)aniline, 4-amino-N-ethyl-N-(β-sulphoethyl)aniline, 4-amino-3-methyl-N-ethyl-N-(β-sulphoethyl)aniline, N-[(4'-amino)phenyl]morpholine, N-[(4'-amino)phenyl]piperidine, 2-β-hydroxyethyl-para-phenylenediamine, 2-fluoro-para-phenylenediamine, 2-carboxy-para-phenylenediamine, 2-sulpho-para-phenylenediamine, 2-isopropyl-para-phenylenediamine, 2-n-propyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine and 2-β-hydroxyethyloxy-para-phenylenediamine, and the addition salts thereof with an acid.

6. Composition according to Claim 5, characterized in that the para-phenylenediamines of formula (II) are chosen from para-phenylenediamine, para-toluylenediamine, 2,6-dimethyl-para-phenylenediamine, 2-β-hydroxyethyl-para-phenylenediamine, 2-n-propyl-para-phenylenediamine, 2-isopropyl-para-phenylenediamine, N-(β-hydroxypropyl)-para-phenylenediamine, N,N-bis(β-hydroxyethyl)-para-phenylenediamine and 4-amino-N-(β-methoxyethyl)aniline, and the addition salts thereof with an acid.

**7.** Composition according to any one of the preceding claims, characterized in that the bis(phenyl)alkylenediamines are chosen from the compounds of following formula (III), and the addition salts thereof with an acid:

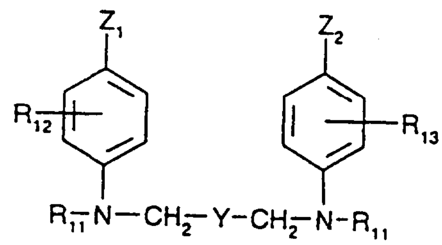

(III)

in which:

$Z_1$ and $Z_2$, which may be identical or different, represent a hydroxyl radical or a radical $NHR_{14}$ in which $R_{14}$ represents a hydrogen atom or a $C_1$-$C_4$ alkyl radical,
$R_{11}$ represents a hydrogen atom, a $C_1$-$C_4$ alkyl, $C_1$-$C_4$ monohydroxyalkyl or $C_2$-$C_4$ polyhydroxyalkyl radical or a $C_1$-$C_4$ aminoalkyl radical in which the amino residue may be substituted,
$R_{12}$ and $R_{13}$, which may be identical or different, represent a hydrogen or halogen atom or a $C_1$-$C_4$ alkyl radical, Y represents a radical taken from the group consisting of the following radicals:
$-(CH_2)_n-$; $-(CH_2)_m-O-(CH_2)_m-$; $-(CH_2)_m-CHOH-(CH_2)_m-$ and

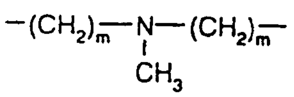 ;

in which n is an integer between 0 and 8 inclusively and m is an integer between 0 and 4 inclusively.

**8.** Composition according to Claim 7, characterized in that the bis(phenyl)alkylenediamines of formula (III) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine and N,N'-bis(ethyl)-N,N'-bis(4'-amino-3'-methylphenyl)ethylenediamine, and the addition salts thereof with an acid.

**9.** Composition according to any one of the preceding claims, characterized in that the addition salts with an acid are chosen from the hydrochlorides, hydrobromides, sulphates and tartrates.

**10.** Composition according to any one of the preceding claims, characterized in that the para-phenylenediamine(s) and/or the bis(phenyl)alkylenediamine(s) represent from 0.0005 to 12 % by weight relative to the total weight of the dye composition.

**11.** Composition according to Claim 10, characterized in that the para-phenylenediamine(s) and/or the bis(phenyl)alkylenediamine(s) represent from 0.005 to 6 % by weight relative to the total weight of the dye composition.

**12.** Composition according to any one of the preceding claims, characterized in that the meta-aminophenols of formula (I) represent from 0.0001 to 10 % by weight relative to the total weight of the dye composition.

**13.** Composition according to Claim 12, characterized in that the meta-aminophenols of formula (I) represent from 0.005 to 5 % by weight relative to the total weight of the dye composition.

**14.** Composition according to any one of the preceding claims, characterized in that the 6-hydroxyindoline and/or the addition salt or salts thereof with an acid represent from 0.0001 to 5 % by weight relative to the total weight of the

dye composition.

15. Composition according to Claim 14, characterized in that the 6-hydroxyindoline and/or the addition salt or salts thereof with an acid represent from 0.005 to 3 % by weight relative to the total weight of the dye composition.

16. Composition according to any one of the preceding claims, characterized in that the appropriate medium for the dyeing (or the support) consists of water or of a mixture of water and at least one organic solvent chosen from $C_1$-$C_4$ lower alkanols, glycerol, glycols and glycol ethers, aromatic alcohols, similar products and mixtures thereof.

17. Composition according to any one of the preceding claims, characterized in that it has a pH of between 3 and 12.

18. Process for dyeing keratin fibres, and in particular human keratin fibres such as the hair, characterized in that at least one dye composition as defined in any one of Claims 1 to 17 is applied to these fibres and in that the colour is developed at acidic, neutral or alkaline pH using an oxidizing agent which is added only at the time of use to the dye composition or which is present in an oxidizing composition that is applied simultaneously or sequentially in a separate manner.

19. Process according to Claim 18, characterized in that the oxidizing agent is chosen from hydrogen peroxide, urea peroxide, alkali metal bromates and persalts such as perborates and persulphates.

20. Multi-compartment device, or multi-compartment dyeing "kit", a first compartment of which contains a dye composition as defined in any one of Claims 1 to 17 and a second compartment of which contains an oxidizing composition.

## Patentansprüche

1. Zusammensetzung zum oxidativen Färben von Keratinfasern und insbesondere von menschlichen Keratinfasern wie dem Haar,
dadurch gekennzeichnet, daß sie in einem zum Färben geeigneten Medium

- mindestens eine Oxidationsbase, die unter den p-Phenylendiaminen und/oder Bisphenylalkylendiaminen ausgewählt ist,
- mindestens ein m-Aminophenol als ersten Kuppler und
- mindestens einen zweiten Kuppler, der unter 6-Hydroxyindolin und seinen Additionssalzen mit einer Säure ausgewählt ist,

enthält.

2. Zusammensetzung nach Anspruch 1, dadurch gekennzeichnet, daß das oder die m-Aminophenole, die als erster Kuppler verwendet werden, unter den Verbindungen der folgenden Formel (I) und den Additionssalzen dieser Verbindungen mit einer Säure ausgewählt sind:

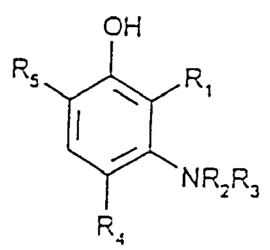

(I)

worin bedeuten:

$R_1$ und $R_4$, die identisch oder voneinander verschieden sind, ein Wasserstoff- oder Halogenatom, $C_{1-4}$-Alkyl, oder $C_{1-4}$-Alkoxy;
$R_2$ ein Wasserstoffatom, $C_{1-4}$-Akyl, $C_{1-4}$-Monohydroxyalkyl, $C_{2-4}$-Polyhydroxyalkyl, $C_{1-4}$-Trifluoralkyl oder Carbamoylmethyl;

$R_3$ ein Wasserstoffatom oder $C_{1-4}$-Alkyl oder $R_3$ bildet mit $R_2$ und dem Stickstoffatom eine fünf- oder sechsgliedrigen Heterocyclus;

$R_5$ ein Wasserstoff- oder Halogenatom, $C_{1-4}$-Alkyl, $C_{1-4}$-Alkoxy, $C_{1-4}$-Monohydroxyalkyl oder $C_{2-4}$-Polyhydroxyalkyl.

**3.** Zusammensetzung nach Anspruch 2, dadurch gekennzeichnet, daß das oder die m-Aminophenole, die als erster Kuppler verwendet werden, ausgewählt sind unter: 3-Aminophenol, 5-Amino-2-methoxyphenol, 5-Amino-4-chlor-2-methylphenol, 5-Amino-2,4-dimethoxyphenol, 5-Amino-2-β-hydroxyethyloxyphenol, 5-N-(β-hydroxyethyl)amino-4-methoxy-2-methylphenol, 5-Amino-4-methoxy-2-methylphenol, 5-N-(γ-hydroxypropyl)amino-2-methylphenol, 5-N-(β-hydroxyethyl)amino-2-methylphenol, 5-Amino-2-methylphenol, 3-N-(carbamoylmethyl)aminophenol, 5-N-(carbamoylmethyl)amino-2-methylphenol, 3-N,N-(dimethyl)aminophenol, 3-N,N-(diethyl)aminophenol, 3-Amino-2,4-dichlorphenol, 3-Amino-4,6-dichlorphenol, 5-Amino-6-chlor-2-methylphenol, 2-Chlor-5-N-(2',2',2'-trifluorethyl) aminophenol, 5-Amino-4-chlor-2-methylphenol, 3-N-(cyclopentyl)aminophenol und den Additionssalzen dieser Verbindungen mit einer Säure.

**4.** Zusammensetzung nach einem der vorhergehenden Ansprüche, dadurch gekennzeichnet, daß die p-Phenylendiamine unter den Verbindungen der folgenden Formel (II) und den Additionssalzen dieser Verbindungen mit einer Säure ausgewählt sind:

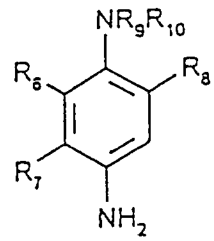

(II)

worin bedeuten:

$R_6$, $R_7$ und $R_8$, die identisch oder voneinander verschieden sind, ein Wasserstoff- oder Halogenatom, $C_{1-4}$-Alkyl, $C_{1-4}$-Alkoxy, Sulfo, Carboxy, $C_{1-4}$-Monohydroxyalky oder $C_{2-4}$-Polyhydroxyalkyl;

$R_9$ und $R_{10}$, die identisch oder voneinander verschieden sind, ein Wasserstoffatom, $C_{1-4}$-Alkyl, $C_{1-4}$-Monohydroxyalkyl, $C_{2-4}$-Polyhydroxyalkyl, $C_{1-4}$-Alkoxy-$C_{1-4}$-alkyl, $C_{1-4}$-Carbamoylalkyl, $C_{1-4}$-Mesylaminoalkyl, $C_{1-4}$-Acetylaminoalkyl, $C_{1-4}$-Ureidoalkyl, $C_{1-4}$-Carbalkoxy-$C_{1-4}$-aminoalkyl, $C_{1-4}$-Sulfoalkyl, $C_{1-4}$-Piperidinoalkyl, $C_{1-4}$-Morpholinoalkyl, Phenyl oder Phenyl, das in p-Stellung mit Amino substituiert ist, oder $R_9$ und $R_{10}$ bilden zusammen mit dem Stickstoffatom, an das sie gebunden sind, einen Piperidino- oder Morpholino-Heterocyclus,

mit der Maßgabe, daß, wenn $R_9$ und $R_{10}$ nicht gleichzeitig Wasserstoff bedeuten, mindestens eine der Gruppen $R_6$ und $R_8$ Wasserstoff ist.

**5.** Zusammensetzung nach Anspruch 4, dadurch gekennzeichnet, daß die p-Phenylendiaminen der Formel (II) ausgewählt sind unter: p-Phenylendiamin, p-Toluylendiamin, 2-chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methylanilin, N,N-Bis-(β-hydroxyethyl)-p-phenylendiamin, 4-Amino-N,N-bis-(β-hydroxyethyl)-3-methylanilin, 4-Amino-3-chlor-N,N-bis-(β-hydroxyethyl)-anilin, 4-Amino-N,N-(ethyl, carbamoylmethyl)-anilin, 4-Amino-3-methyl-N,N-(ethyl, carbamoylmethyl)-anilin, 4-Amino-N,N-(ethyl, β-piperidinoethyl)-anilin, 4-Amino-3-methyl-N,N-(ethyl, β-piperidinoethyl)-anilin, 4-Amino-N,N-(ethyl, β-morpholinoethyl)-anilin, 4-Amino-3-methyl-N,N-(ethyl, β-morpholinoethyl)-anilin, 4-Amino-N,N-(ethyl, β-acetylaminoethyl)-anilin, 4-Amino-N-(β-methoxyethyl)-anilin, 4-Amino-3-methyl-N,N-(ethyl, β-acetylaminoethyl)-anilin, 4-Amino-N,N-(ethyl, β-mesylaminoethyl)-anilin, 4-Amino-3-methyl-N,N-(ethyl, β-mesylaminoethyl)-anilin, 4-Amino-N,N-(ethyl, β-sulfoethyl)-anilin, 4-Amino-3-methyl-N,N-(ethyl, β-sulfoethyl)-anilin, N-[(4'-Amino)phenyl]-morpholin, N-[(4'-Amino)phenyl]-piperidin, 2-β-Hydroxyethyl-p-phenylendiamin, 2-Fluor-p-phenylendiamin, 2-Carboxy-p-phenylendiamin, 2-Sulfo-p-phenylendiamin, 2-Isopropyl-p-phenylendiamin, 2-n-Propyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin und den Additionssalzen dieser Verbindungen mit einer Säure.

6. Zusammensetzung nach Anspruch 5, dadurch gekennzeichnet, daß die p-Phenylendiamine der Formel (II) ausgewählt sind unter: p-Phenylendiamin, p-Toluylendiamin, 2,6-Dimethyl-p-phenylendiamin, 2-β-Hydroxyethyl-p-phenylendiamin, 2-n-Propyl-p-phenylendiamin, 2-Isopropyl-p-phenylendiamin, N-(β-Hydroxypropyl)-p-phenylendiamin, N,N-Bis-(β-hydroxyethyl)-p-phenylendiamin, 4-Amino-N-(β-methoxyethyl)-anilin und den Additionssalzen dieser Verbindungen mit einer Säure.

7. Zusammensetzung nach einem der vorhergehenden Ansprüche, dadurch gekennzeichnet, daß die Bisphenylalkylendiamine unter den Verbindungen der folgenden Formel (III) und den Additionssalzen dieser Verbindungen mit einer Säure ausgewählt sind:

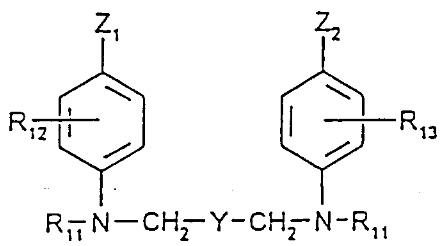

(III)

worin bedeuten:

$Z_1$ und $Z_2$, die identisch oder voneinander verschieden sind, Hydroxy oder $NHR_{14}$, wobei $R_{14}$ Wasserstoff oder $C_{1-4}$-Alkyl bedeutet,
$R_{11}$ Wasserstoff, $C_{1-4}$-Alkyl, $C_{1-4}$-Monohydroxyalkyl, $c_{2-4}$-Polyhydroxyalkyl oder $C_{1-4}$-Aminoalkyl, wobei die Aminoguppe substituiert sein kann,
$R_{12}$ und $R_{13}$, die identisch oder voneinander verschieden sind, ein Wasserstoff- oder Halogenatom oder $C_{1-4}$-Alkyl, Y eine Gruppe, die unter den folgenden Gruppen ausgewählt ist:
$-(CH_2)_n-$ ; $-(CH_2)_m-O-(CH_2)_m-$ ; $-(CH_2)_m-CHOH-(CH_2)_m-$ und

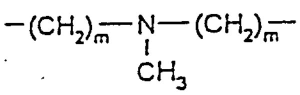 ;

wobei n Null oder eine ganze Zahl von 1 bis einschließlich 8 und m Null oder eine ganze Zahl von 1 bis einschließlich 4 bedeutet.

8. Zusammensetzung nach Anspruch 7, dadurch gekennzeichnet, daß die Bisphenylalkylendiamine der Formel (III) ausgewählt sind unter: N,N'-Bis-(β-hydroxyethyl)-N,N'-bis-(4'-aminophenyl)-1,3-diamino-propanol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-bis-(4-methylaminophenyl)-tetramethylendiamin, N,N'-bis-(ethyl)-N,N'-bis-(4'-amino-3'-methylphenyl)-ethylendiamin und den Additionssalzen dieser Verbindungen mit einer Säure.

9. Zusammensetzung nach einem der vorhergehenden Ansprüche, dadurch gekennzeichnet, daß die Additionssalze mit einer Säure unter den Hydrochloriden, Hydrobromiden, Sulfaten und Tartraten ausgewählt sind.

10. Zusammensetzung nach einem der vorhergehenden Ansprüche, dadurch gekennzeichnet, daß das oder die p-Phenylendiamine und/oder das oder die Bisphenylalkylendiamine 0,0005 bis 12 Gew.-% des Gesamtgewichts der Färbemittelzusammensetzung ausmachen.

11. Zusammensetzung nach Anspruch 10, dadurch gekennzeichnet, daß das oder die p-Phenylendiamine und/oder

das oder die Bisphenylalkylendiamine 0,005 bis 6 Gew.-% des Gesamtgewichts der Färbemittelzusammensetzung ausmachen.

12. Zusammensetzung nach einem der vorhergehenden Ansprüche, dadurch gekennzeichnet, daß das oder die m-Aminophenole der Formel (I) 0,0001 bis 10 Gew.-% des Gesamtgewichts der Färbemittelzusammensetzung ausmachen.

13. Zusammensetzung nach Anspruch 12, dadurch gekennzeichnet, daß das oder die m-Aminophenole der Formel (I) 0,005 bis 5 Gew.-% des Gesamtgewichts der Färbemittelzusammensetzung ausmachen.

14. Zusammensetzung nach einem der vorhergehenden Ansprüche, dadurch gekennzeichnet, daß das 6-Hydroxyindolin und/oder seine Additionssalze mit einer Säure 0,0001 bis 5 Gew.-% des Gesamtgewichts der Färbemittelzusammensetzung ausmachen.

15. Zusammensetzung nach Anspruch 14, dadurch gekennzeichnet, daß das 6-Hydroxyindolin und/oder seine Additionssalze mit einer Säure 0,005 bis 3 Gew.-% des Gesamtgewichts der Färbemittelzusammensetzung ausmachen.

16. Zusammensetzung nach einem der vorhergehenden Ansprüche, dadurch gekennzeichnet, daß das zum Färben geeignete Medium aus Wasser oder einem Gemisch aus Wasser und mindestens einem organischen Lösungsmittel besteht, das unter den niederen Alkanolen mit 1 bis 4 Kohlenstoffatomen, Glycerin, Glykolen und Glykolethern, aromatischen Alkoholen, analogen Produkten und deren Gemischen ausgewählt ist.

17. Zusammensetzung nach einem der vorhergehenden Ansprüche, dadurch gekennzeichnet, daß sie einen pH-Wert im Bereich von 3 bis 12 aufweist.

18. Verfahren zum Färben von Keratinfasern und insbesondere von menschlichen Keratinfasern, wie dem Haar, dadurch gekennzeichnet, daß auf die Fasern eine Färbemittelzusammensetzung nach einem der Ansprüche 1 bis 17 aufgetragen und die Farbe bei einem sauren, neutralen oder alkalischen pH-Wert mit einem Oxidationsmittel entwickelt wird, das bei der Anwendung zu der Färbemittelzusammensetzung gegeben wird oder das in einer oxidierenden Zusammensetzung vorliegt, die gleichzeitig oder getrennt davon anschließend aufgetragen wird.

19. Verfahren nach Anspruch 18, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

20. Vorrichtung mit mehreren Abteilungen oder "Kit" zum Färben mit mehreren Abteilungen, wobei eine erste Abteilung eine Färbemittelzusammensetzung nach einem der Ansprüche 1 bis 17 und eine zweite Abteilung eine oxidierende Zusammensetzung enthält.